# EUROPEAN PATENT APPLICATION

(11) **EP 2 204 192 A1**
(43) Date of publication of application: **07.07.2010**
(21) Application number: 08837483.0
(22) Date of filing: 10.10.2008
(51) Int. Cl.: A61K 45/00, A61K 31/513, A61P 1/04, A61P 13/10, A61P 19/02, A61P 29/00, A61P 43/00, C07D 403/06

(54) **PROPHYLACTIC OR THERAPEUTIC AGENT FOR INFLAMMATORY DISEASE COMPRISING THYMIDINE PHOSPHORYLASE INHIBITOR AS ACTIVE INGREDIENT**

(30) Priority: 11.10.2007 JP 2007265276
(71) Applicant: Taiho Pharmaceutical Co., Ltd., Tokyo 101-8444 (JP)
(72) Inventor: SAKAMOTO, Kazuki, Tokushima-shi Tokushima 771-0132 (JP); SUGIMOTO, Yoshikazu, Tokyo 101-8444 (JP)
(74) Representative: Hartz, Nikolai
(86) International application number: PCT/JP2008/002860
(87) International publication number: WO 2009/047903

(57) **Abstract**

To provide an excellent agent for prevention or treatment of an inflammatory disease.

The agent for prevention or treatment of an inflammatory disease contains a thymidine phosphorylase inhibitor as an active ingredient, wherein when the thymidine phosphorylase inhibitor is 5-chloro-6-(2-iminopyrrolidin-1-yl)methyl-2,4(1H,3H)-pyrimidinedione or a salt thereof, the inflammatory disease is an inflammatory disease other than inflammatory bowel disease.

## Description

### Field of the Invention

The present invention relates to a novel agent for prevention or treatment of inflammatory diseases.

### Background of the Invention

Inflammatory reaction is a type of biological defense reaction occurring when a harmful substance invades the body, and a series of such a biological reactions against the substance are collectively referred to as inflammation. In an acute stage, the inflammatory reaction involves microvasodilation, promotion of vascular (microvein) permeability through diastasis of endothelial space, tissue destruction, etc.; while in a chronic stage, degeneration and fibrosis of tissue occur, leading to repair of inflammatory tissue. When the inflammatory reaction, a mechanism for elimination of foreign body, excessively proceeds, the reaction causes a body-damaging pathological condition, which requires to be treated.

Hitherto, among various anti-inflammatory drugs, steroidal anti-inflammatory drugs, acidic non-steroidal anti-inflammatory drugs, basic non-steroidal anti-inflammatory drugs (NSAIDs) have generally been employed. A steroidal anti-inflammatory drug binds to a receptor present in cytoplasm and is incorporated into a cell nucleus, inducing biosynthesis of lipocortin. Lipocortin is known to exhibit anti-inflammatory effect through suppression of various chemical mediators; e.g., an inhibitory effect of a cyclooxygenase pathway via inhibition of phospholipase A2, and an inhibitory effect of a lipoxygenase pathway concomitant with inhibition of leukotriene B4. Steroidal anti-inflammatory drugs exhibit remarkable inhibitory effect to a wide range of inflammatory diseases and are effective for autoimmune diseases. However, use of a steroidal anti-inflammatory drug has been confirmed to often involve strong adverse side effects such as aggravation of infections, digestive ulcer, osteoporosis, suppression of ACTH secretion from the hypothalamus or the like, and abnormal water-electrolyte metabolism. Therefore, the dose, in particular the maintenance dose, of such a steroidal anti-inflammatory drug is desired to be minimized as far as possible. Meanwhile, NSAIDs exhibit anti-inflammatory effect via inhibition of biosynthesis of prostaglandin through suppression of activity of cyclooxygenase. Thus, NSAIDs also have antipyretic analgesic effect. Generally, side effect of NSAIDs is relatively mild as compared with steroidal anti-inflammatory drugs. However, in some cases, NSAIDs involve adverse side effects such as gastrointestinal disorders, kidney disorders, liver disorders, blood and hematopoietic system disorders, central nervous system disorders, and skin toxicity. Therefore, in order to suppress the adverse side effects, combination use of additional drugs such as a gastric-mucosa-protecting agent may be required.

As described above, some conventional anti-inflammatory agents cause adverse side effects due to their effect mechanisms. Therefore, development of a novel anti-inflammatory agent exhibiting a new effect mechanism should be of great clinical value.

### Summary of the Invention

### Problems to be Solved by the Invention

An object of the present invention is to provide a therapeutic agent capable of effectively treating inflammatory diseases.

### Means for Solving the Problems

The present inventors have extensively studied for solving the above problems, and have found that a uracil compound represented by the following formula (1), which is a thymidine phosphorylase inhibitor, or a salt thereof, is useful for treatment of rheumatoid arthritis, cystitis, acute gastritis, chronic gastritis, or hepatitis. The inventors have also found that the uracil compound or a salt thereof inhibits production of interleukin 6 (hereinafter referred to as IL-6), which plays a main role in inflammatory reaction. These findings indicate that the uracil compound or a salt thereof is applicable to the treatment not only of the aforementioned diseases but also of other inflammatory diseases. The present invention has been accomplished on the basis of these findings.

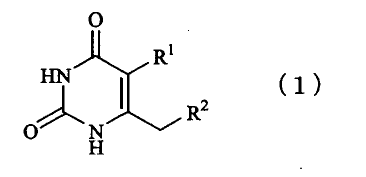

(wherein R¹ represents a chlorine atom, a bromine atom, an iodine atom, a cyano group, or a lower alkyl group; and R² represents a 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; a lower alkylamidino group; an amino group in which a hydrogen atom bonded to the nitrogen atom is optionally substituted with a lower alkyl group; -CH₂N(R^{a})R^{b} group (wherein R^{a} and R^{b}, which are identical to or different from each other, each represent a hydrogen atom or a lower alkyl group, or R^{a} and R^{b} may form a pyrrolidine ring with the nitrogen atom to which they are bonded); -NH-(CH₂)ₘ-Z group (wherein Z represents an amino group in which a hydrogen atom bonded to the nitrogen atom is optionally substituted with a lower alkyl group, or a cyano group; and m is an integer of 0 to 3); -NR^{c}(CH₂)ₙ-OH group (wherein R^{c} represents a hydrogen atom or a lower alkyl group, and n is a natural number of 1 to 4); -X-Y group (wherein X represents S or NH; and Y represents a 2-imidazolin-2-yl, 2-imidazolyl, 1-methylimidazol-2-yl, 1,2,4-triazol-3-yl, 2-pyrimidyl, or 2-benzimidazolyl group which is optionally substituted with a lower alkyl group); or a ureido or thioureido group in which a hydrogen atom bonded to the nitrogen atom is optionally substituted with a lower alkyl group).

Accordingly, the present invention provides an agent for prevention or treatment of an inflammatory disease containing a thymidine phosphorylase inhibitor as an active ingredient, wherein when the thymidine phosphorylase inhibitor is 5-chloro-6-(2-iminopyrrolidin-1-yl)methyl-2,4(1H,3H)-pyrimidinedione or a salt thereof, the inflammatory disease is an inflammatory disease other than inflammatory bowel disease.
The present invention also provides use of a thymidine phosphorylase inhibitor for production of a drug for prevention or treatment of inflammatory disease, wherein when the thymidine phosphorylase inhibitor is 5-chloro-6-(2-iminopyrrolidin-1-yl)methyl-2,4(1H,3H)-pyrimidinedione or a salt thereof, the inflammatory disease is an inflammatory disease other than inflammatory bowel disease.
The present invention also provides a method for prevention or treatment of inflammatory disease, **characterized in that** the method comprises administering, to a subject in need thereof, an effective amount of a thymidine phosphorylase inhibitor, wherein when the thymidine phosphorylase inhibitor is 5-chloro-6-(2-iminopyrrolidin-1-yl)methyl-2,4(1H,3H)-pyrimidinedione or a salt thereof, the inflammatory disease is an inflammatory disease other than inflammatory bowel disease.

Thymidine phosphorylase is an enzyme which reversibly converts thymidine to thymine and 2-deoxyribose-1-phosphate, and is known to be involved in DNA synthesis. High expression of thymidine phosphorylase is observed in various solid tumors (mainly gastric cancer and colorectal cancer). The expression level of thymidine phosphorylase is positively correlated with angiogenesis, invasion, metastasis, etc., clearly indicating that thymidine phosphorylase is a prognosis factor (Cancer Sci. 2004 Nov; 95(11): 851-7, Int. J. Clin. Oncol. 2006 Aug; 11(4): 297-302). Regarding other pathological conditions, increased expression of thymidine phosphorylase is also reported in disease site of patients suffering inflammatory diseases (glomerulonephritis, interstitial cystitis, chronic rheumatoid arthritis, and inflammatory bowel disease) (Nephron Clin. Pract. 2006; 102(3-4): c133-42, J. Urol. 2002 Jan; 167(1): 347-51, Clin. Chim. Acta. 1993 Sep; 217(1): 1-4, J. Gastroenterol. 2003; 38(3): 229-37). However, the role of thymidine phosphorylase in the aforementioned inflammatory diseases has not been completely elucidated. A thymidine phosphorylase inhibitor has never been known to inhibit production of IL-6.

### Effects of the Invention

According to the present invention, inflammatory diseases such as rheumatoid arthritis, cystitis, acute gastritis, chronic gastritis, and hepatitis can be effectively treated.

### Brief Description of the Drawings

[Fig. 1] Graphs showing an effect for improvement of a bladder function through TPI administration on hydrochloric-acid-induced cystitis model rats.
[Fig. 2] A graph showing an effect through TPI administration to collagen arthritis model mice.
[Fig. 3] A graph showing a suppression of mucosal damage (lesion length) of hydrochloric-acid-induced gastritis model rats through administration of TPI.
[Fig. 4] Graphs showing effects through TPI administration on AST and ALT on concanavalin-A-induced hepatitis model mice.
[Fig. 5] A graph showing an effect of thymidine on production of IL-6 in human luekemia cell-derived U937, and IL-6 production inhibitory effect of a compound exhibiting TP inhibitory effect.

### Detailed description of the Invention

The thymidine phosphorylase inhibitor employed on the present invention is not particularly limited, so long as the inhibitor is a substance capable of lowering the enzymatic activity of thymidine phosphorylase. Either a substance inhibiting the enzymatic activity of thymidine phosphorylase or a substance suppressing expression of thymidine phosphorylase may be used.
Examples of the substance which suppresses the expression level of thymidine phosphorylase include an antisense nucleic acid complementary to a transcription product derived from a thymidine phosphorylase gene or a portion thereof; a nucleic acid which exhibits an effect of inhibiting expression of a thymidine phosphorylase gene through RNAi effect; and a nucleic acid which exhibits a ribozyme activity for specifically cleaving a transcription product derived from a thymidine phosphorylase gene. The aforementioned nucleic acids may be produced through a generally known method. For example, the antisense nucleic acid complementary to a transcription product of a thymidine phosphorylase gene or a portion thereof may be produced through a method described in Cancer Res. 2004, Oct 15; 64(20): 7526-32, and the nucleic acid which exhibits an effect of inhibiting expression of a thymidine phosphorylase gene through RNAi effect may be produced through a method described in International Publication WO 2004/111237 (pamphlet).

Examples of the substance inhibiting the enzymatic activity of thymidine phosphorylase include a low-molecule compound or an antibody (or a fragment thereof) which binds to thymidine phosphorylase. Examples of the low-molecule compound include uracil compounds represented by the following formula (1) and salts thereof, TUPI (5-chloro-6-aminouracil), 7-deazaxanthine, KIN-59 (5'-O-tritylinosine), and SHetA2 ({[(4-nitrophenyl)amino][(2,2,4,4-tetrametyl thiochroman-6-yl)amino]methane-1-thione}). Among them, uracil compounds represented by the following formula (1) and salts thereof are preferred.

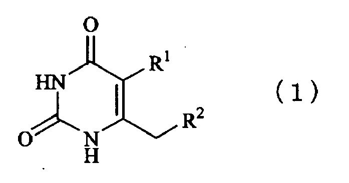

(wherein R¹ represents a chlorine atom, a bromine atom, an iodine atom, a cyano group, or a lower alkyl group; and R² represents a 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; a lower alkylamidino group; an amino group in which a hydrogen atom bonded to the nitrogen atom is optionally substituted with a lower alkyl group; -CH₂N(R^{a})R^{b} group (wherein R^{a} and R^{b}, which are identical to or different from each other, each represent a hydrogen atom or a lower alkyl group, or R^{a} and R^{b} may form a pyrrolidine ring with the nitrogen atom to which they are bonded); -NH-(CH₂)ₘ-Z group (wherein Z represents an amino group in which a hydrogen atom bonded to the nitrogen atom is optionally substituted with a lower alkyl group, or a cyano group; and m is an integer of 0 to 3); -NR^{c}(CH₂)ₙ-OH group (wherein R^{c} represents a hydrogen atom or a lower alkyl group, and n is a natural number of 1 to 4); -X-Y group (wherein X represents S or NH; and Y represents a 2-imidazolin-2-yl, 2-imidazolyl, 1-methylimidazol-2-yl, 1,2,4-triazol-3-yl, 2-pyrimidyl, or 2-benzimidazolyl group which is optionally substituted with a lower alkyl group); or a ureido or thioureido group in which a hydrogen atom bonded to the nitrogen atom is optionally substituted with a lower alkyl group).

The uracil compound represented by formula (1) or a salt thereof will be described below.
The uracil compound represented by formula (1) or a salt thereof is known to have an inhibitory effect activity on thymidine phosphorylase, and to be employed as an antitumor agent (JP-B-3088757), a cancer-metastasis-inhibiting agent (JP-B-3088758), a side-effect-reducing agent (JP-A-2000-273044), or an anti-HIV agent (JP-A-2001-131075). However, the uracil compound represented by formula (1) or a salt thereof has never been known to be able to inhibit production of IL-6 or to be employed in the treatment of inflammatory disease.

In formula (1), the lower alkyl group represented by R¹ or R² is, for example, a C1 to C4 linear or branched alkyl group. Specific examples include methyl, ethyl, n-propyl, isopropyl, n-butyl, isobutyl, sec-butyl, and tert-butyl group. Of these, methyl group is preferred.

Examples of the a 4- to 8-membered heterocyclic group which is represented by R² and which has 1 to 3 nitrogen atoms include 1-azetidinyl, 1-pyrrolidinyl, 2-pyrrolin-1-yl, 3-pyrrolin-1-yl, 1-pyrrolyl, 1-pyrazolidinyl, 2-pyrazolin-1-yl, 3-pyrazolin-1-yl, 4-pyrazolin-1-yl, 1-pyrazolyl, 1-imidazolidinyl, 2-imidazolin-1-yl, 3-imidazolin-1-yl, 4-imidazolin-1-yl, 1-imidazolyl, 1,2,3-triazol-1-yl, 1,2,4-triazol-1-yl, piperidino, 1-piperazyl, morpholino, 1-perhydroazepinyl, and 1-perhydroazocinyl group. The heterocycle of the heterocyclic group may have one or two substituents. Examples of such substituents include a lower alkyl group, an imino group, a hydroxyl group, a hydroxymethyl group, a methanesulfonyloxy group, an amino group, and a nitro group. Specific examples of the heterocyclic group which may have such a substituent include 1-azetidinyl, 1-pyrrolidinyl, 2,5-dimethylpyrrolidin-1-yl, 2-iminopyrrolidin-1-yl, 3-hydroxypyrrolidin-1-yl, 2-hydroxymethylpyrrolidin-1-yl, 3-methanesulfonyloxypyrrolidin-1-yl, 3-aminopyrrolidin-1-yl, 2-pyrrolin-1-yl, 3-pyrrolin-1-yl, 2-imino-3-pyrrolin-1-yl, 1-pyrrolyl, 1-pyrazolidinyl, 2-methylpyrazolidin-1-yl, 4-iminopyrazolidin-1-yl, 2-pyrazolin-1-yl, 3-pyrazolin-1-yl, 2-methyl-3-pyrazolin-1-yl, 5-imino-3-pyrazolin-1-yl, 4-pyrazolin-1-yl, 2-methyl-4-pyrazolin-1-yl, 3-imino-4-pyrazolin-1-yl, 1-pyrazolyl, 1-imidazolidinyl, 3-methylimidazolidin-1-yl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 3-imidazolin-1-yl, 4-imidazolin-1-yl, 3-methyl-4-imidazolin-1-yl, 2-imino-4-imidazolin-1-yl, 2-imino-3-methyl-4-imidazolin-1-yl, 2-imino-3-ethyl-4-imidazolin-1-yl, 2-imino-3-isopropyl-4-imidazolin-1-yl, 1-imidazolyl, 2-methylimidazol-1-yl, 2-nitroimidazol-1-yl, 4-nitroimidazol-1-yl, 1,2,3-triazol-1-yl, 1,2,4-triazol-1-yl, 3-nitro-1,2,4-triazol-1-yl, piperidino, 1-piperazyl, 4-methylpiperazin-1-yl, morpholino, 1-perhydroazepinyl, and 1-perhydroazocinyl group. The preferred examples include 1-azetidinyl, 1-pyrrolidinyl, 2-iminopyrrolidin-1-yl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, and 1-imidazolyl group.

In the amidinothio group in which a hydrogen atom bonded to the nitrogen atom of R² is optionally substituted with a lower alkyl group, 1 to 3 of the three hydrogen atoms each bonded to the nitrogen of the amidino group is optionally substituted with the aforementioned lower alkyl groups. Particularly preferred are an amidinothio group, an N¹-methylamidinothio group, and an N¹,N²-dimethylamidinothio group.

In the guanidino group in which a hydrogen atom bonded to the nitrogen atom is optionally substituted with a lower alkyl group or a cyano group, 1 to 4 of the four hydrogen atoms of the guanidino group is optionally substituted with the aforementioned lower alkyl group or a cyano group. Particularly preferred are a 1-guanidino group, a 1-methylguanidino group, a 3-methylguanidino group, a 2,3-dimethylguanidino group, and a 2-cyano-3-methylguanidino group.

The lower alkylamidino group is formed of an amidino group to which the aforementioned lower alkyl group is bonded. Among such groups, an acetamidino group is preferred.

In the amino group in which a hydrogen atom bonded to the nitrogen atom is optionally substituted with a lower alkyl group, 1 or 2 of the two hydrogen atoms of the amino group is optionally substituted with the aforementioned lower alkyl group. Among such groups, an amino group, an N-methylamino group, an N,N-dimethylamino group, an N-ethylamino group, an N,N-diethylamino group, an N-propylamino group, and an N-isopropylamino group are preferred.

Among -CH₂N(R^{a})R^{b} groups, an N-methylaminomethyl group, an N,N-dimethylaminomethyl group, and a 1-pyrrolidinylmethyl group are preferred.

Among -NH-(CH₂)ₘ-Z groups, an N,N-dimethylhydrazino group, an N-(2-aminoethyl)amino group, an N-(2-(N,N-dimethyl)aminoethyl)amino group, an N-(3-aminopropyl)amino group, and an N-(2-cyanoethyl)amino group are preferred.

Among -NR^{c}(CH₂)ₙ-OH groups, an N-(2-hydroxyethyl)-N-methylamino group, an N-(3-hydroxypropyl)amino group, and an N-(4-hydroxybutyl)amino group are preferred.

Among -X-Y groups, a 2-imidazoline-2-thio group, a 2-imidazolin-2-amino group, an imidazol-2-thio group, a 1-methylimidazole-2-thio group, a 1,2,4-triazole-3-thio group, a pyrimidine-2-thio group, and a benzimidazole-2-thio group are preferred.

Among the ureido or thioureido groups in which a hydrogen atom bonded to the nitrogen atom is optionally substituted with a lower alkyl group, a ureido group and a 3-methylthioureido group are preferred.

In formula (1), the group R² is preferably a 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; or a lower alkylamidino group.

Specific examples of preferred groups R² include 1-azetidinyl, 1-pyrrolidinyl, 2-iminopyrrolidin-1-yl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, amidinothio, N¹-methylamidinothio, N¹,N²-dimethylamidinothio, 1-guanidino, 1-methylguanidino, 3-methylguanidino, 2,3-dimethylguanidino, and acetamidino group.

In the uracil compound represented by formula (1), preferably, R¹ is a chlorine atom, a bromine atom, or a cyano group; and R² is a 1-pyrrolidinyl group, a 1-azetidinyl group, a 2-iminopyrrolidin-1-yl group, a 2-iminoimidazolidin-1-yl group, a 1-imidazolyl group, an amidinothio group, or a 1-guanidino group.

The salt of the uracil compound represented by formula (1) is not particularly limited, so long as the salt is pharmaceutically acceptable. Among the salts, an acid-added salt produced by use of a pharmaceutically acceptable acid is preferred. Examples of the acid-added salt include salts with an inorganic acid such as hydrochloric acid, sulfuric acid, phosphoric acid, or hydrobromic acid; and salts with an organic acid such as oxalic acid, maleic acid, fumaric acid, malic acid, tartaric acid, citric acid, benzoic acid, acetic acid, p-toluenesulfonic acid, or methanesulfonic acid. Of these, hydrochloric acid salts and p-toluenesulfonic acid salts are preferred.

Specific examples of particularly preferred uracil compounds represented by formula (1) and salts thereof include 5-chloro-6-(2-iminopyrrolidin-1-yl)methyl-2,4(1H,3H)-pyrimidinedione hydrochloride and 5-chloro-6-(2-iminopyrrolidin-1-yl) tosylate.

The uracil compound represented by formula (1) or a salt thereof may be produced through a generally known production method, for example, a method described in JP-B-3088757.

The antibody serving as the thymidine phosphorylase inhibitor of the present invention may be a monoclonal or polyclonal antibody, so long as the antibody binds to thymidine phosphorylase. Antibody fragments such as Fab, Fab' , F(ab')₂, Fv, and scFv may also be used. Such antibody fragments may be produced through, for example, digestion of an antibody with papain or the like, or a procedure including construction of a gene encoding the antibody fragment, incorporation of the gene into an expression vector, and expression of the gene in appropriate host cells.

The source of the antibody serving as the thymidine phosphorylase inhibitor of the present invention is not particularly limited, a mouse antibody, a rat antibody, a rabbit antibody, a sheep antibody, a human antibody, a chimera antibody, or a humanized antibody may be appropriately used.

The antibody serving as the thymidine phosphorylase inhibitor of the present invention may be produced through a generally known immunological method, for example, a method described in Biol. Pharm. Bull. 1996 Nov; 19(11): 1407-11.

As mentioned in the below-described Examples, the thymidine phosphorylase inhibitor of the present invention is useful for prevention or treatment of rheumatoid arthritis, cystitis, acute gastritis, chronic gastritis, and hepatitis. Furthermore, the thymidine phosphorylase inhibitor of the present invention can inhibit production of IL-6, which is known to play a key role in inflammatory reaction. Therefore, the inhibitor of the present invention can prevent or treat not only the aforementioned diseases but also the other various inflammatory diseases.

The "inflammatory disease" of the present invention is not particularly limited, so long as the disease involves inflammatory reaction in which TP is expressed. Examples of the inflammatory disease include rheumatoid arthritis, cystitis (interstitial cystitis, acute or chronic cystitis, hemorrhagic cystitis, radiation cystitis, or cystophthisis), acute or chronic gastritis, systemic juvenile idiopathic arthritis, Castleman's disease, systemic erythematosus, ankylosing spondylitis, scabies, scleroderma, Sjoegren's syndrome, atopic dermatitis, peritonitis, asthma, chronic bronchitis, chronic obstructive lung disease, pneumonia, allergic rhinitis, allergic conjunctivitis, chronic lung inflammatory disease, multiple sclerosis, polyarteritis nodosa, Wegener's granuloma, atherosclerosis, transplant rejection reaction, juvenile type I diabetes, glomerulonephritis, chronic nephritis, uveitis, Behcet's syndrome, and hepatitis (including acute hepatitis and chronic hepatitis). Among them, rheumatoid arthritis, cystitis (interstitial cystitis, acute or chronic cystitis, hemorrhagic cystitis, radiation cystitis, or cystophthisis), acute or chronic gastritis, and hepatitis are preferred, with rheumatoid arthritis, interstitial cystitis, acute gastritis, and hepatitis being particularly preferred.

As used herein, the term "treatment" of inflammatory disease encompasses curing the inflammatory disease and also mitigation of conditions involved in the inflammatory disease.

The prophylactic or therapeutic agent of the present invention for inflammatory disease may be produced in a variety of dosage forms using the thymidine phosphorylase inhibitor of the present invention and pharmaceutically acceptable carrier through a generally known drug preparation method. The dosage form is not particularly limited, and examples thereof include oral preparations such as tablets, coated tablets, pills, powders, granules, capsules, solutions, suspensions, and emulsions; and parenteral preparations such as injections and suppositories.

In forming tablets, examples of the carrier which may be employed in the invention include excipients such as lactose, sucrose, sodium chloride, glucose, urea, starch, calcium carbonate, kaolin, crystalline cellulose, and silicic acid; binders such as water, ethanol, propanol, corn starch, simple syrup, glucose solution, starch solution, gelatin solution, carboxymethyl cellulose, shellac, methyl cellulose, hydroxypropyl cellulose, hydroxypropylmethyl cellulose, potassium phosphate, and polyvinylpyrrolidone; disintegrating agents such as dry starch, sodium alginate, agar powder, laminaran powder, sodium bicarbonate, calcium carbonate, polyoxyethylene sorbitan fatty acid esters, sodium lauryl sulfate, stearic acid monoglyceride, and lactose; disintegration inhibitors such as sucrose, stearic acid, cacao butter, and hydrogenated oil; absorption-accelerating agents such as quaternary ammonium salts and sodium lauryl sulfate; humectants such as glycerol and starch; adsorbents such as starch, lactose, kaolin, bentonite, and colloidal silica; and lubricants such as purified talc, stearic acid salts, boric acid powder, and polyethylene glycol. The tablets, as required, may be prepared as coated tablets using usual coatings, such as sugar-coated tablets, gelatin-coated tablets, enteric coated tablets, film-coated tablets, double-layer tablets, and multi-layer tablets.

In forming pills, examples of the carriers to be employed include excipients such as glucose, lactose, starch, cacao butter, hardened vegetable oil, kaolin, and talc; binders such as powdered acacia, powdered tragacanth, gelatin, and ethanol; and disintegrating agents such as laminaran and agar. Capsules are prepared through a routine method comprising the step of mixing an active ingredient with the aforementioned various carriers and filling appropriate capsules such as hard gelatin capsules or soft capsules with the mixture.

In preparing oral liquid preparations, for example, internal liquid, syrups or elixirs may be produced through a routine method by use of a flavoring agent, a buffer, a stabilizer, a corrigent, etc. Examples of the flavoring agent include sucrose, orange peel, citric acid, and tartaric acid, and examples of the buffer include sodium citrate. Examples of the stabilizer include tragacanth, acacia, and gelatin.
In forming suppositories, examples of the carrier to be employed include polyethylene glycol, cacao butter, higher alcohol, higher alcohol esters, gelatin, and semi-synthetic glyceride.

In preparing injections, injection liquids, injection emulsions, and injection suspensions are sterilized, and are preferably isotonic to blood. Examples of the diluent to be used in preparing injections include water, aqueous lactic acid, ethyl alcohol, propylene glycol, macrogol, ethoxylated isostearyl alcohol, polyoxyethylenated isostearyl alcohol, and polyoxyethylene sorbitan fatty acid esters.

In this case, the pharmaceutical preparation may contain sodium chloride, glucose, or glycerol in an amount sufficient for preparing isotonic solution. The preparation may also contain generally employed other additives such as a solubilizing agent, a buffer, and a soothing agent. If required, the aforementioned preparation may further contain a colorant, a preservative, a perfume, a flavor, a sweetener, etc. and other pharmaceutical products.

The method of administering the prophylactic or therapeutic agent of the present invention for inflammatory disease is not particularly limited, and the method is appropriately selected depending on, for example, the dosage form, the age, sex, or other conditions of a patient, or the severity of symptoms of a patient. For example, tablets, pills, granules, capsules, solutions, suspensions, and emulsions are orally administered. While, injections are intravenously administered alone or mixtures thereof with a general fluid replacements such as glucose or amino acid. If required, they are administered alone intraarterially, intramuscularly, intracutaneously, subcutaneously, or intraperitoneally. Suppositories are used through rectal administration.

The dose of the active ingredient of agent for prevention or treatment of inflammatory disease of the present invention is appropriately selected depending on, for example, the dosage form, the age, sex, or other conditions of a patient, or the severity of symptoms of a patient. When the uracil compound represented by formula (1) or a salt thereof is employed as an active ingredient, the dose thereof is about 0.01 to about 100 mg/kg/day, preferably about 0.1 to about 100 mg/kg/day.

The present invention is described in more detail by way of examples, which should not be construed as limiting the invention thereto.

### Examples

### Example 1 (Effect on hydrochloric-acid-induced cystitis model rat)

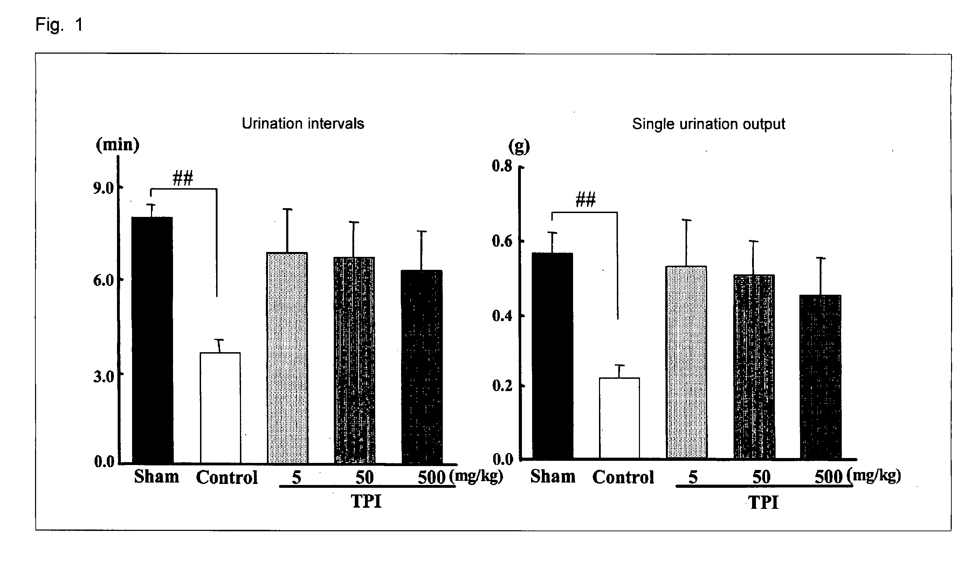

To rats having hydrochloric-acid-induced cystitis serving as a cystitis model, 5-chloro-6-(2-iminopyrrolidin-1-yl)methyl-2,4(1H,3H)-pyrimidinedione hydrochloride (hereinafter abbreviated as "TPI") (5, 50, or 100 mg/kg) was orally administered continuously for seven days, and the effect of TPI on bladder functions was evaluated.
Specifically, 10-week-old SD rats were divided into groups (n = 6) on the basis of body weight. To each of the rats of the control group and TPI administration groups, 0.4N HCl (0.2 mL) was intravesically administered once to the bladder under pentobarbital anesthesia (50 mg/kg, i.p.), to thereby induce a cystitis model. To a Sham group, saline (0.2 mL) was intravesically administered. From the day following the induction of cystitis model, distilled water for injection (5 mL/kg) was administered to the Sham and control groups, while distilled water for injection (5 mL/kg) containing TPI (5, 50, or 100 mg/kg) was administered to the TPI administration groups. In each case, the liquid was administered by forced oral administration once a day continuously for seven days. On the day following the final administration day, bladder functions (urination intervals and single urination output) of the rats of each group were evaluated through cystometry under anesthesia in accordance with a method described in BJU Int. 2007 Oct; 100(4): 935-9. The results are shown in Fig. 1.
As shown in Fig. 1, shortening of urination interval, decreases in single urination output and effective vesical capacity, and an increase in residual urine were observed in the control group. In the TPI administration groups, evaluations of these items were improved in response to the administration amount. Thus, TPI was found to be a useful compound for the treatment of interstitial cystitis.

### Example 2 (Effect on hydrochloric-acid-induced rat interstitial cystitis model)

To rats having hydrochloric-acid-induced cystitis serving as an interstitial cystitis model, TPI (50 or 500 mg/kg) was orally administered continuously for seven days, and the effect of TPI on pathological histological improvement of the bladder tissue was evaluated.
Specifically, 10-week-old SD rats were divided into groups (n = 6) on the basis of body weight. To each of the rats of a control group and TPI administration groups, 0.4N HCl (0.2 mL) was intravesically administered once to the bladder under pentobarbital anesthesia (50 mg/kg, i.p.), to thereby induce a cystitis model. To a Sham group, saline (0.2 mL) was intravesically administered. From the day following the induction of cystitis model, distilled water for injection (5 mL/kg) was administered to the Sham and control groups, while distilled water for injection (5 mL/kg) containing TPI (50 or 500 mg/kg) was administered to the TPI administration groups. In each case, the liquid was orally administered continuously once a day for seven days. On the day following the final administration day, the bladder was removed from each of the rats under ether anesthesia and washed with saline. Subsequently, the washed bladder was fixed with 20% buffered formalin and 10% buffered formalin, followed by HE staining. Histological changes of the bladder were observed. The results are shown in Table 1. Histological changes were evaluated in terms of thickening and proliferation of transitional epithelium, infiltration of mast cells and eosinocytes into the lamina propria, occurrence of edema in the lamina propria, infiltration of mast cells and eosinocytes into the tunica muscularis, and occurrence of edema in the tunica muscularis, with the three ratings (0, 1, and 2).

TPI at a dose of 50 and 500 mg/kg ameliorates thickening of transitional epithelium, edema in the lamina propria, and swelling of fibroblasts and infiltration of inflammatory cells in the lamina propria, as well as interstitial edema in the tunica muscularis and infiltration of inflammatory cells in the tunica muscularis. Thus, TPI was found to be a useful compound for the treatment of interstitial cystitis.

**[Table 1]**

| Site/Findings | Group Sample No. | Sham | | | Control | | | TAS-111 (50) | | | TAS-111 (500) | | |
|---|---|---|---|---|---|---|---|---|---|---|---|---|---|
| | | + | ++ | +++ | + | ++ | +++ | + | ++ | +++ | + | ++ | +++ |
| Entire bladder | | | | | | | | | | | | | |
| Thickening/proliferation of transitional epithelium | | 0 | 0 | 0 | 0 | 1 | 2 | 1 | 2 | 0 | 1 | 0 | 0 |

| Lamina propria | | | | | | | | | | | | | |
|---|---|---|---|---|---|---|---|---|---|---|---|---|---|
| Infiltration of mast cells/eosinocytes | | 0 | 0 | 0 | 1 | 1 | 1 | 1 | 2 | 0 | 2 | 0 | 0 |
| Edema | | 0 | 0 | 0 | 1 | 2 | 0 | 0 | 2 | 0 | 0 | 1 | 0 |

| Tunica muscularis | | | | | | | | | | | | | |
|---|---|---|---|---|---|---|---|---|---|---|---|---|---|
| Infiltration of mast cells/eosinocytes | | 0 | 0 | 0 | 2 | 1 | 0 | 2 | 0 | 0 | 0 | 0 | 0 |
| Edema | | 0 | 0 | 0 | 2 | 1 | 0 | 2 | 0 | 0 | 0 | 0 | 0 |

### Example 3 (Effect of TPI administration on collagen arthritis model mouse)

TPI (250 or 500 mg/kg) was orally administered to collagen arthritis mice (i.e., rheumatic model) continuously for 14 days, and the effect of TPI on edema of the limbs of each mouse was evaluated.
Arthritis was induced in DBA/A1 mice by type II collagen induction, and the mice were employed as a rheumatoid arthritis model. Specifically, type II collagen derived from bovine joint (K42, product of Collagen Gijyutsu Kensyukai) (200 µg) was suspended in Freund's complete adjuvant (DIFCO) (100 µL), and on day 1, the suspension was intradermally administered to the back of 8-week-old mice (DBA/1JN mice, Charles River Laboratories Japan Inc.). On day 21, type II collagen derived from bovine joint (K42) (200 µg) suspended in Freund's complete adjuvant (100 µL) was intradermally administered to the tail portion of each mice, to thereby induce an arthritis model.
To the TPI administration groups, TPI (250 or 500 mg/kg/day) was orally administered continuously once a day during a period from day 21 to day 40. To the control, a solvent (0.5% aqueous HPMC) was administered instead of TPI according to the same administration schedule as employed in the TPI administration groups. As a reference drug, prednisolone (3 mg/kg/day) was administered according to the same administration schedule as employed in the TPI administration groups.

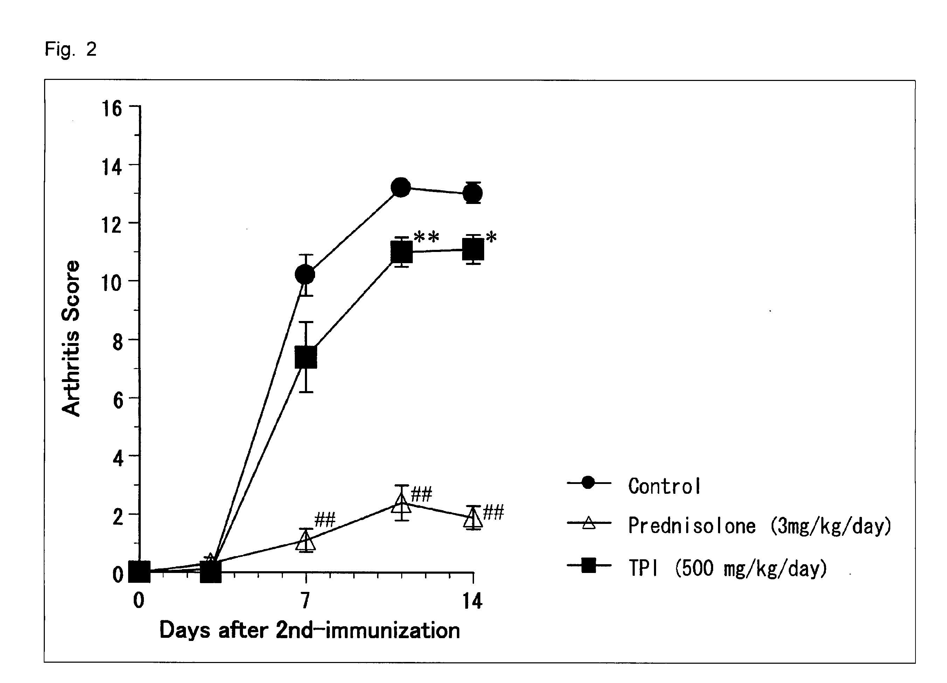

The arthritis condition was evaluated on day 42; i.e., the day following the final administration day. The following scores of the arthritis condition of one limb were employed: no change (0), flare or swelling in one digit (1), flare or swelling in two or more digits or backside flare (2), backside swelling (3), and swelling in all digits and swelling in the ankle (wrist) (4). The scores of four limbs were summed, and the total score was used for evaluation (highest: 16). The results are shown in Fig. 2.

The change of arthritis scores indicates the highest level of edema in a period from 11 to 14 days after second immunization. In contrast, the TPI administration (500 mg/kg) group exhibited significant suppression of the arthritis scores in a period from 11 to 14 days after second immunization. Thus, TPI was found to be a useful compound for the treatment of rheumatoid arthritis.

### Example 4 (Effect on hydrochloric-acid-induced acute gastritis model rat)

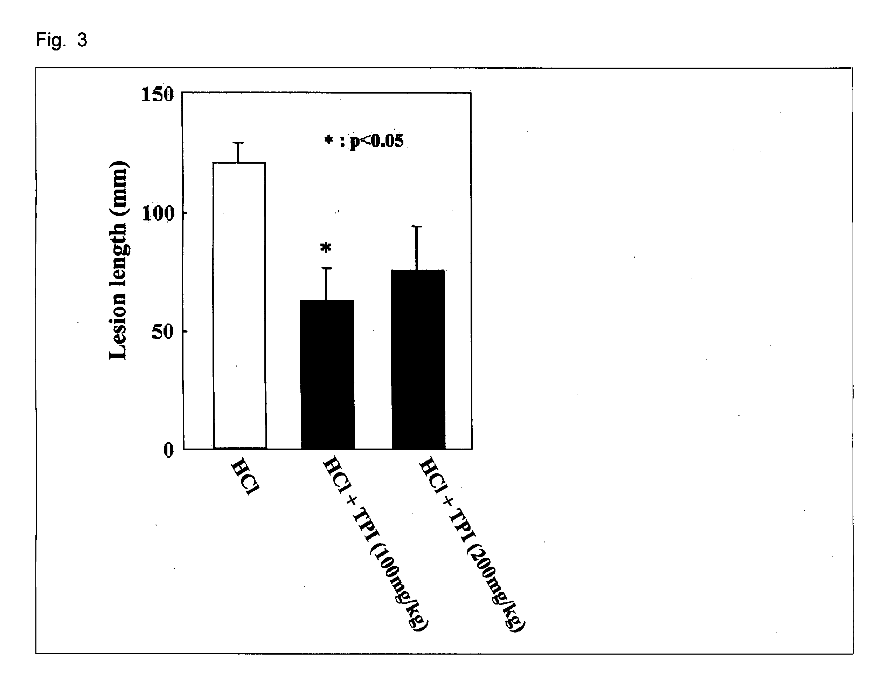

TPI (100 or 200 mg/kg) was orally single-administered to rats in which ulcer had been induced by hydrochloric acid (i.e., an acute gastritis model), and the preventive effect of TPI on mucosal damage was evaluated.
Specifically, 7-week-old female SD rats (Charles River Laboratories Japan Inc.) were fasted for about 18 hours. To the TPI administration groups, TPI (100 or 200 mg/kg) was orally administered, and after 30 minutes 0.6N HCl (5 mL/kg) was orally administered. To the control group, only 0.6N HCl (5 mL/kg) was orally administered. One hour after administration of hydrochloric acid, rats were euthanized through , and the stomach was removed from each rat. The tissue thereof was fixed with 1% formaline buffer, and the stomach was cut through the greater curvature. The longer diameter (mm) of a lesion was measured by means of an electronic caliper, and the total of the diameters was employed as an index for evaluating gastric mucosal damage. The results are shown in Fig. 3.
In the control group, gastric mucosal damage of 120.4 ± 8.5 mm was observed. In the TPI (100 mg/kg) administration group, gastric mucosal damage was significantly reduced to 63.0 ± 14.3 mm, and in the TPI (200 mg/kg) administration group, gastric mucosal damage was reduced to 76.1 ± 18.3 mm. The difference therebetween is not significant. Thus, TPI was found to be a useful compound for the treatment of acute gastritis.

### Example 5 (Effect on hepatitis model)

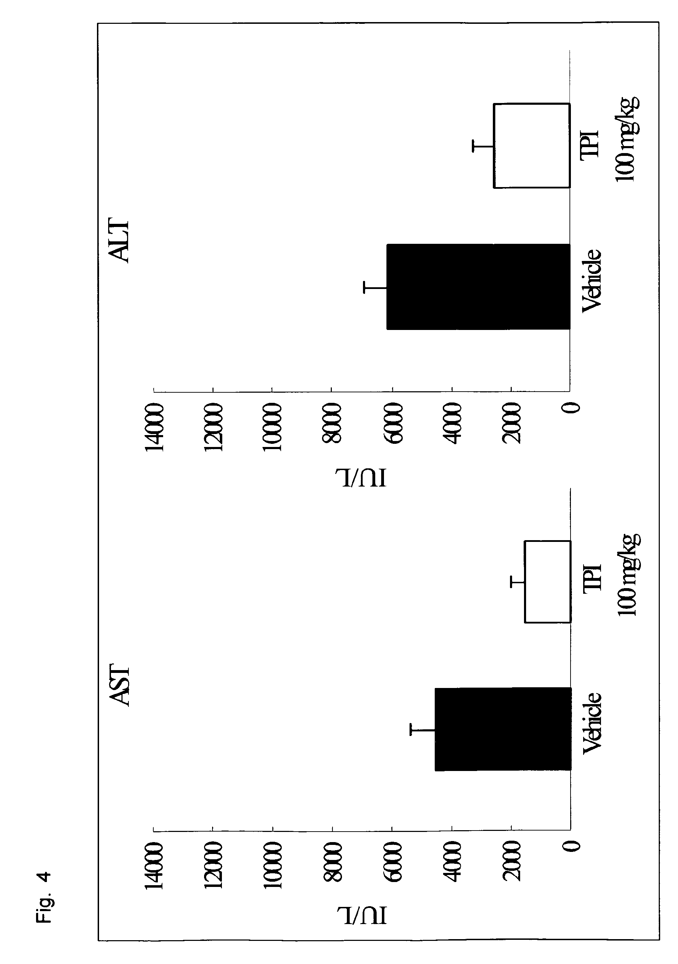

TPI (100 mg/kg) was orally single-administered to rats in which hepatitis had been induced by concanavalin A (i.e., a hepatitis model), and the inhibitory effect of TPI on hepatitis was evaluated.
Specifically, 6-week-old female C57BL/6NCrlCrlj mice (Charles River Laboratories Japan Inc.) were employed. To the TPI administration group, TPI (100 mg/kg) was orally administered, and after 30 minutes 2 mg/mL concanavalin A (150 µL/animal) was intravenously administered through the tail vein. To the control group, only a vehicle was orally administered instead of TPI. Twenty-four hours after the administration of concanavalin A, blood was sampled from each mouse through the abdominal inferior vena cava under diethyl ether anesthesia. The serum ALT level (IU/L) and AST level (IU/L) were determined as index for liver disorders. The results are shown in Fig. 4.
In the control group, AST and ALT increased to 4,506 ± 864 and 6,130 ± 800, respectively. In the TPI administration (100 mg/kg) group, AST and ALT significantly decreased to 1,540 ± 477 and 2,538 ± 755, respectively. Thus, TPI is a useful compound for the treatment of hepatitis.

### Example 6 (Effect of thymidine on production of IL-6 by human luekemia cell-derived U937, and effect of compound having TP inhibitory effect on inhibition of IL-6 production)

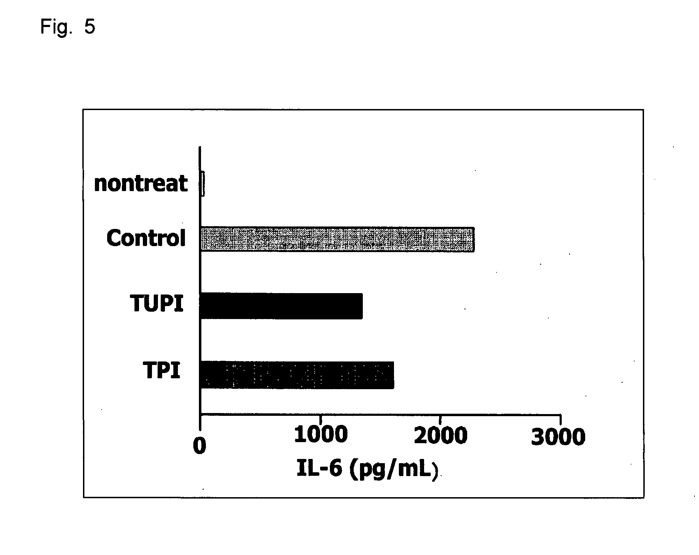

Human monocytes cell line U937 were cultured using a 10% FBS RPMI 1640 medium (SIGMA-ALDRICH) at 37°C under 5% CO₂. Using a 24-well plate, U937 cells (1 × 10⁵) were cultured for 24 hours in a medium containing 10 ng/mL phorbol-12-myristate-13-acetate (inflammatory substance, hereinafter abbreviated to as PMA) and 10 µmol/L thymidine in order for induction of inflammatory reaction. Twenty-four hours later, the medium was changed to fresh medium. To the drug administration group, TUPI or TPI was added. Forty-eight hours after the addition, the IL-6 level of each culture supernatant was determined by means of BioPlex (Bio-Rad Laboratories, Inc.). The results are shown in Fig. 5.
The result is that, as compared with the control group, the drug (TUPI or TPI) administration groups exhibited more significant suppression of IL-6 level. Thus, TPI was found to have an IL-6 production inhibitory effect.

## Claims

**1.** An agent for prevention or treatment of an inflammatory disease containing an uracil compound represented by formula (1) or a salt thereof, wherein when the uracil compound represented by formula (1) or a salt thereof is 5-chloro-6-(2-iminopyrrolidin-1-yl)methyl-2,4(1H,3H)-pyrimidinedione or a salt thereof, the inflammatory disease is an inflammatory disease other than inflammatory bowel disease: 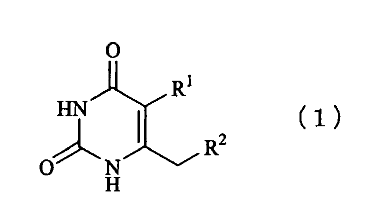 (wherein R¹ represents a chlorine atom, a bromine atom, an iodine atom, a cyano group, or a lower alkyl group; and R² represents a 4-to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; a lower alkylamidino group; an amino group in which a hydrogen atom bonded to the nitrogen atom is optionally substituted with a lower alkyl group; -CH₂N(R^{a})R^{b} group (wherein R^{a} and R^{b}, which are identical to or different from each other, each represent a hydrogen atom or a lower alkyl group, or R^{a} and R^{b} may form a pyrrolidine ring with the nitrogen atom to which they are bonded); -NH-(CH₂)ₘ-Z group (wherein Z represents an amino group in which a hydrogen atom bonded to the nitrogen atom is optionally substituted with a lower alkyl group, or a cyano group; and m is an integer of 0 to 3); -NR^{c}(CH₂)ₙ-OH group (wherein R^{c} represents a hydrogen atom or a lower alkyl group, and n is a natural number of 1 to 4); -X-Y group (wherein X represents S or NH; and Y represents a 2-imidazolin-2-yl, 2-imidazolyl, 1-methylimidazol-2-yl, 1,2,4-triazol-3-yl, 2-pyrimidyl, or 2-benzimidazolyl group which is optionally substituted with a lower alkyl group); or a ureido or thioureido group in which a hydrogen atom bonded to the nitrogen atom is optionally substituted with a lower alkyl group).

**4.** The agent for prevention or treatment according to claim 1, wherein the group R² in formula (1) is a 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; or a lower alkylamidino group.

**5.** The agent for prevention or treatment according to claim 1 or 4, wherein the 4- to 8-membered heterocyclic group which has 1 to 3 nitrogen atoms, represented by R² in formula (1), is 1-azetidinyl, 1-pyrrolidinyl, 2-pyrrolin-1-yl, 3-pyrrolin-1-yl, 1-pyrrolyl, 1-pyrazolidinyl, 2-pyrazolin-1-yl, 3-pyrazolin-1-yl, 4-pyrazolin-1-yl, 1-pyrazolyl, 1-imidazolidinyl, 2-imidazolin-1-yl, 3-imidazolin-1-yl, 4-imidazolin-1-yl, 1-imidazolyl, 1,2,3-triazol-1-yl, 1,2,4-triazol-1-yl, piperidino, 1-piperazyl, morpholino, 1-perhydroazepinyl, or 1-perhydroazocinyl group.

**6.** The agent for prevention or treatment according to any one of claims 1, 4 and 5, wherein the 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms, represented by R² in formula (1), is 1-azetidinyl, 1-pyrrolidinyl, 2,5-dimethylpyrrolidin-1-yl, 2-iminopyrrolidin-1-yl, 3-hydroxypyrrolidin-1-yl, 2-hydroxymethylpyrrolidin-1-yl, 3-methanesulfonyloxypyrrolidin-1-yl, 3-aminopyrrolidin-1-yl, 1-pyrrolyl, 2-pyrazolin-1-yl, 1-pyrazolyl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, 2-methylimidazol-1-yl, 2-nitroimidazol-1-yl, 4-nitroimidazol-1-yl, 1,2,3-triazol-1-yl, 1,2,4-triazol-1-yl, 3-nitro-1,2,4-triazol-1-yl, piperidino, 4-methylpiperazin-1-yl, morpholino, 1-perhydroazepinyl, or 1-perhydroazocinyl group.

**7.** The agent for prevention or treatment according to any one of claims 1 and 4 to 6, wherein the group R² in formula (1) is 1-azetidinyl, 1-pyrrolidinyl, 2-iminopyrrolidin-1-yl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, amidinothio, N¹-methylamidinothio, N¹,N²-dimethylamidinothio, 1-guanidino, 1-methylguanidino, 3-methylguanidino, 2,3-dimethylguanidino, or acetamidino group.

**8.** The agent for prevention or treatment according to any one of claims 1 and 4 to 7, wherein the group R¹ in formula (1) is a chlorine atom, a bromine atom, or a cyano group; and the group R² in formula (1) is a 1-pyrrolidinyl group, a 1-azetidinyl group, a 2-iminopyrrolidin-1-yl group, a 2-iminoimidazolidin-1-yl group, a 1-imidazolyl group, an amidinothio group, or a 1-guanidino group.

**9.** An agent for prevention or treatment according to any one of claims 1 and 4 to 8, wherein the uracil compound or a salt thereof is selected from the group consisting of 5-chloro-6-(1-pyrrolidinylmethyl)uracil, 5-bromo-6-(1-pyrrolidinylmethyl)uracil, 5-chloro-6-(1-azetidinylmethyl)uracil, 5-chloro-6-(1-(2-iminopyrrolidinyl)methyl)uracil hydrochloride, 5-bromo-6-(1-(2-iminopyrrolidinyl)methyl)uracil hydrochloride, 5-cyano-6-(1-(2-iminopyrrolidinyl)methyl)uracil, 5-chloro-6-(1-(2-iminoimidazolidinyl)methyl)uracil, 5-bromo-6-(1-(2-iminoimidazolidinyl)methyl)uracil, 5-chloro-6-(1-imidazolylmethyl)uracil hydrochloride, 2-(5-chlorouracil-6-ylmethyl)isothiourea hydrochloride, 2-(5-cyanouracil-6-ylmethyl)isothiourea hydrochloride, and 5-chloro-6-(1-guanidino)methyluracil hydrochloride.

**10.** The agent for prevention or treatment according to any one of claims 1 and 4 to 9, wherein the inflammatory disease is rheumatoid arthritis, cystitis, acute gastritis, chronic gastritis, or hepatitis.

**11.** An use of an uracil compound represented by formula (1) or a salt thereof for production of an agent for prevention or treatment of an inflammatory disease, wherein when the uracil compound represented by formula (1) or a salt thereof is 5-chloro-6-(2-iminopyrrolidin-1-yl)methyl-2,4(1H,3H)-pyrimidinedione or a salt thereof, the inflammatory disease is an inflammatory disease other than inflammatory bowel disease: 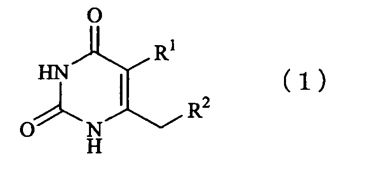 (wherein R¹ represents a chlorine atom, a bromine atom, an iodine atom, a cyano group, or a lower alkyl group; and R² represents a 4-to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; a lower alkylamidino group; an amino group in which a hydrogen atom bonded to the nitrogen atom is optionally substituted with a lower alkyl group; -CH₂N(R^{a})R^{b} group (wherein R^{a} and R^{b}, which are identical to or different from each other, each represent a hydrogen atom or a lower alkyl group, or R^{a} and R^{b} may form a pyrrolidine ring with the nitrogen atom to which they are bonded); -NH-(CH₂)ₘ-Z group (wherein Z represents an amino group in which a hydrogen atom bonded to the nitrogen atom is optionally substituted with a lower alkyl group, or a cyano group; and m is an integer of 0 to 3); -NR^{c}(CH₂)ₙ-OH group (wherein R^{c} represents a hydrogen atom or a lower alkyl group, and n is a natural number of 1 to 4); -X-Y group (wherein X represents S or NH; and Y represents a 2-imidazolin-2-yl, 2-imidazolyl, 1-methylimidazol-2-yl, 1,2,4-triazol-3-yl, 2-pyrimidyl, or 2-benzimidazolyl group which is optionally substituted with a lower alkyl group); or a ureido or thioureido group in which a hydrogen atom bonded to the nitrogen atom is optionally substituted with a lower alkyl group).

**14.** The use according to claim 11, wherein the group R² in formula (1) is a 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; or a lower alkylamidino group.

**15.** The use according to claim 11 or 14, wherein the 4- to 8-membered heterocyclic group which has 1 to 3 nitrogen atoms, represented by R² in formula (1), is 1-azetidinyl, 1-pyrrolidinyl, 2-pyrrolin-1-yl, 3-pyrrolin-1-yl, 1-pyrrolyl, 1-pyrazolidinyl, 2-pyrazolin-1-yl, 3-pyrazolin-1-yl, 4-pyrazolin-1-yl, 1-pyrazolyl, 1-imidazolidinyl, 2-imidazolin-1-yl, 3-imidazolin-1-yl, 4-imidazolin-1-yl, 1-imidazolyl, 1,2,3-triazol-1-yl, 1,2,4-triazol-1-yl, piperidino, 1-piperazyl, morpholino, 1-perhydroazepinyl, or 1-perhydroazocinyl group.

**16.** The use according to any one of claims 11, 14 and 15,
wherein the 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms, represented by R² in formula (1), is 1-azetidinyl, 1-pyrrolidinyl, 2,5-dimethylpyrrolidin-1-yl, 2-iminopyrrolidin-1-yl, 3-hydroxypyrrolidin-1-yl, 2-hydroxymethylpyrrolidin-1-yl, 3-methanesulfonyloxypyrrolidin-1-yl, 3-aminopyrrolidin-1-yl, 1-pyrrolyl, 2-pyrazolin-1-yl, 1-pyrazolyl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, 2-methylimidazol-1-yl, 2-nitroimidazol-1-yl, 4-nitroimidazol-1-yl, 1,2,3-triazol-1-yl, 1,2,4-triazol-1-yl, 3-nitro-1,2,4-triazol-1-yl, piperidino, 4-methylpiperazin-1-yl, morpholino, 1-perhydroazepinyl, or 1-perhydroazocinyl group.

**17.** The use according to any one of claims 11 and 14 to 16,
wherein the group R² in formula (1) is 1-azetidinyl, 1-pyrrolidinyl, 2-iminopyrrolidin-1-yl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, amidinothio, N¹-methylamidinothio, N¹,N²-dimethylamidinothio, 1-guanidino, 1-methylguanidino, 3-methylguanidino, 2,3-dimethylguanidino, or acetamidino group.

**18.** The use according to any one of claims 11 and 14 to 17,
wherein the group R¹ in formula (1) is a chlorine atom, a bromine atom, or a cyano group; and the group R² in formula (1) is a 1-pyrrolidinyl group, a 1-azetidinyl group, a 2-iminopyrrolidin-1-yl group, a 2-iminoimidazolidin-1-yl group, a 1-imidazolyl group, an amidinothio group, or a 1-guanidino group.

**19.** The use according to any one of claims 11 and 14 to 18,
wherein the uracil compound or a salt thereof is selected from among 5-chloro-6-(1-pyrrolidinylmethyl)uracil, 5-bromo-6-(1-pyrrolidinylmethyl)uracil, 5-chloro-6-(1-azetidinylmethyl)uracil, 5-chloro-6-(1-(2-iminopyrrolidinyl)methyl)uracil hydrochloride, 5-bromo-6-(1-(2-iminopyrrolidinyl)methyl)uracil hydrochloride, 5-cyano-6-(1-(2-iminopyrrolidinyl)methyl)uracil, 5-chloro-6-(1-(2-iminoimidazolidinyl)methyl)uracil, 5-bromo-6-(1-(2-iminoimidazolidinyl)methyl)uracil, 5-chloro-6-(1-imidazolylmethyl)uracil hydrochloride, 2-(5-chlorouracil-6-ylmethyl)isothiourea hydrochloride, 2-(5-cyanouracil-6-ylmethyl)isothiourea hydrochloride, and 5-chloro-6-(1-guanidino)methyluracil hydrochloride.

**20.** The use according to any one of claims 11 and 14 to 19,
wherein the inflammatory disease is rheumatoid arthritis, cystitis,
acute gastritis, chronic gastritis, or hepatitis.

**21.** A method for prevention or treatment of an inflammatory disease, **characterized in that** the method comprises administering, to a subject in need thereof, an effective amount of an uracil compound represented by formula (1) or a salt thereof, wherein when the uracil compound represented by formula (1) or a salt thereof is 5-chloro-6-(2-iminopyrrolidin-1-yl)methyl-2,4(1H,3H)-pyrimidinedione or a salt thereof, the inflammatory disease is an inflammatory disease other than inflammatory bowel disease: 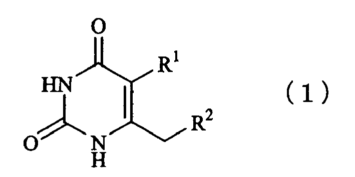 (wherein R¹ represents a chlorine atom, a bromine atom, an iodine atom, a cyano group, or a lower alkyl group; and R² represents a 4-to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; a lower alkylamidino group; an amino group in which a hydrogen atom bonded to the nitrogen atom is optionally substituted with a lower alkyl group; -CH₂N(R^{a})R^{b} group (wherein R^{a} and R^{b}, which are identical to or different from each other, each represent a hydrogen atom or a lower alkyl group, or R^{a} and R^{b} may form a pyrrolidine ring with the nitrogen atom to which they are bonded); -NH-(CH₂)ₘ-Z group (wherein Z represents an amino group in which a hydrogen atom bonded to the nitrogen atom is optionally substituted with a lower alkyl group, or a cyano group; and m is an integer of 0 to 3); -NR^{c}(CH₂)ₙ-OH group (wherein R^{c} represents a hydrogen atom or a lower alkyl group, and n is a natural number of 1 to 4); -X-Y group (wherein X represents S or NH; and Y represents a 2-imidazolin-2-yl, 2-imidazolyl, 1-methylimidazol-2-yl, 1,2,4-triazol-3-yl, 2-pyrimidyl, or 2-benzimidazolyl group which is optionally substituted with a lower alkyl group); or a ureido or thioureido group in which a hydrogen atom bonded to the nitrogen atom is optionally substituted with a lower alkyl group) or a salt thereof.

**24.** The method according to claim 21, wherein the group R² in formula (1) is a 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms; an amidinothio group in which a hydrogen atom bonded to the nitrogen atom is optionally substituted with a lower alkyl group; a guanidino group in which a hydrogen atom bonded to the nitrogen atom is optionally substituted with a lower alkyl group or with a cyano group; or a lower alkylamidino group.

**25.** The method according to claim 21, wherein the 4- to 8-membered heterocyclic group which has 1 to 3 nitrogen atoms, represented by R² in formula (1), is 1-azetidinyl, 1-pyrrolidinyl,
2-pyrrolin-1-yl, 3-pyrrolin-1-yl, 1-pyrrolyl, 1-pyrazolidinyl, 2-pyrazolin-1-yl, 3-pyrazolin-1-yl, 4-pyrazolin-1-yl, 1-pyrazolyl, 1-imidazolidinyl, 2-imidazolin-1-yl, 3-imidazolin-1-yl, 4-imidazolin-1-yl, 1-imidazolyl, 1,2,3-triazol-1-yl, 1,2,4-triazol-1-yl, piperidino, 1-piperazyl, morpholino, 1-perhydroazepinyl, or 1-perhydroazocinyl group.

**26.** The method according to claim 21, wherein the 4- to 8-membered heterocyclic group which is optionally substituted with a lower alkyl group, an imino group, a hydroxyl group, a hydroxymethyl group, a methanesulfonyloxy group, an amino group, or a nitro group and which has 1 to 3 nitrogen atoms, represented by R² in formula (1), is 1-azetidinyl, 1-pyrrolidinyl, 2,5-dimethylpyrrolidin-1-yl, 2-iminopyrrolidin-1-yl, 3-hydroxypyrrolidin-1-yl, 2-hydroxymethylpyrrolidin-1-yl, 3-methanesulfonyloxypyrrolidin-1-yl, 3-aminopyrrolidin-1-yl, 1-pyrrolyl, 2-pyrazolin-1-yl, 1-pyrazolyl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, 2-methylimidazol-1-yl, 2-nitroimidazol-1-yl, 4-nitroimidazol-1-yl, 1,2,3-triazol-1-yl, 1,2,4-triazol-1-yl, 3-nitro-1,2,4-triazol-1-yl, piperidino, 4-methylpiperazin-1-yl, morpholino, 1-perhydroazepinyl, or 1-perhydroazocinyl group.

**27.** The method according to claim 21, wherein the group R² in formula (1) is 1-azetidinyl, 1-pyrrolidinyl, 2-iminopyrrolidin-1-yl, 2-iminoimidazolidin-1-yl, 2-imino-3-methylimidazolidin-1-yl, 2-imino-3-ethylimidazolidin-1-yl, 2-imino-3-isopropylimidazolidin-1-yl, 2-imidazolin-1-yl, 2-imino-3-methyl-4-imidazolin-1-yl, 2-imino-3-ethyl-4-imidazolin-1-yl, 1-imidazolyl, amidinothio, N¹-methylamidinothio, N¹,N²-dimethylamidinothio, 1-guanidino, 1-methylguanidino, 3-methylguanidino, 2,3-dimethylguanidino, or acetamidino group.

**28.** The method according to claim 21, wherein the group R¹ in formula (1) is a chlorine atom, a bromine atom, or a cyano group; and the group R² in formula (1) is a 1-pyrrolidinyl group, a 1-azetidinyl group, a 2-iminopyrrolidin-1-yl group, a 2-iminoimidazolidin-1-yl group, a 1-imidazolyl group, an amidinothio group, or a 1-guanidino group.

**29.** The method according to claim 21, wherein the uracil compound or a salt thereof is selected from the group consisting of 5-chloro-6-(1-pyrrolidinylmethyl)uracil, 5-bromo-6-(1-pyrrolidinylmethyl)uracil, 5-chloro-6-(1-azetidinylmethyl)uracil, 5-chloro-6-(1-(2-iminopyrrolidinyl)methyl)uracil hydrochloride, 5-bromo-6-(1-(2-iminopyrrolidinyl)methyl)uracil hydrochloride, 5-cyano-6-(1-(2-iminopyrrolidinyl)methyl)uracil, 5-chloro-6-(1-(2-iminoimidazolidinyl)methyl)uracil, 5-bromo-6-(1-(2-iminoimidazolidinyl)methyl)uracil, 5-chloro-6-(1-imidazolylmethyl)uracil hydrochloride, 2-(5-chlorouracil-6-ylmethyl)isothiourea hydrochloride, 2-(5-cyanouracil-6-ylmethyl)isothiourea hydrochloride, and 5-chloro-6-(1-guanidino)methyluracil hydrochloride.

**30.** The method according to any one of claims 21 and 24 to 29, wherein the inflammatory disease is rheumatoid arthritis, cystitis, acute gastritis, chronic gastritis, or hepatitis.
